# EUROPEAN PATENT SPECIFICATION

(11) **EP 2 997 988 B1**
(45) Date of publication and mention of the grant of the patent: **26.09.2018**
(21) Application number: 14861997.6
(22) Date of filing: 27.01.2014
(51) Int. Cl.: A61M 5/145, A61M 5/142, A61M 5/00, A61B 5/00

(54) **SINGLE NEEDLE INTEGRATED ARTIFICIAL PANCREAS**
EINZELNADELINTEGRIERTE KÜNSTLICHE BAUCHSPEICHELDRÜSE
PANCRÉAS ARTIFICIEL À AIGUILLE UNIQUE INTÉGRÉE

(30) Priority: 12.11.2013 CN 201310560946
(43) Date of publication of application: 23.03.2016
(73) Proprietor: Medtrum Technologies Inc., Shanghai 201203 (CN)
(72) Inventor: YANG, Cuijun, Shanghai 201203 (CN)
(74) Representative: J A Kemp
(86) International application number: PCT/CN2014/071526
(87) International publication number: WO 2015/070543

(56) References cited:
- WO-A1-2009/066288
- WO-A1-2013/053535
- WO-A2-2010/023666
- CN-A- 1 859 943
- CN-A- 1 973 768
- CN-A- 101 925 372
- CN-A- 102 946 923
- CN-A- 102 970 927
- CN-U- 202 682 463
- US-A- 5 569 186
- US-A1- 2008 086 042
- US-A1- 2008 221 509
- US-A1- 2008 275 384

## Description

### TECHNICAL FIELD

The present disclosure generally relates to the field of medical appliance, specifically to an artificial pancreas, and more particularly, to a single needle integrated artificial pancreas.

### BACKGROUND

As is known to all, diabetes is a metabolic disease caused by pancreatic dysfunction. Diabetes is a lifelong disease which can't be cured by current medical technology. The only way to control the initiation and development of diabetes and its complications is stabilizing glucose. A normal pancreas may automatically monitor the changes of glucose, and may automatically secrete insulin required. "Real time dynamic glucose monitoring system" indicates a device that can real-timely and dynamically monitor the changes of glucose by using a glucose sensor implanted in the subcutaneous tissue of a patient. "Insulin pump" indicates a device that can continuously and accurately deliver insulin to the subcutaneous tissue of the patient for 24 hours by using an indwelling cannula implanted in the subcutaneous tissue of the patient. "Artificial pancreas" indicates a device that can real-timely and dynamically monitor the change of glucose, and can continuously and accurately deliver insulin to the subcutaneous tissue of the patient for 24 hours. A conventional artificial pancreas usually consists of two sets of devices, a real time dynamic glucose monitoring system and an insulin pump system. Therefore, the patient is required to wear at least two sets of discrete components, a glucose sensor and an indwelling cannula, at the same time, when the diabetes is being treated by the conventional artificial pancreas. Considering the comfort level of patients when they are wearing these components, the two discrete components are both made of slender and soft medical polymer material. Because of the special nature of the material and the shape of the two discrete components, they both need to be put into the subcutaneous tissue of a patient with a help of a puncture needle with a certain rigidity to puncture the skin of patients. Thereafter, the needle is pulled out, leaving the two components in the subcutaneous tissue. For using the above two components, the patient must have subcutaneous tissue punctures in two positions of his/her body. Obviously, the more the places to be punctured are required, the more complex the installation is, and the greater the chance of infection may be. The patient must also use large volume auxiliary installation device such as install catapults of glucose sensor and indwelling cannula, etc. The glucose sensor and the indwelling cannula have similar processes of puncture and indwelling, and similar mechanical structures for realizing these processes. Moreover, the glucose sensor and the indwelling cannula are also the same in the aspects such as area of action on body, disposable using, aseptic production, etc.

Moreover, the temperature of human skin surface can greatly affect the insulin peak action time. The longer the insulin peak action time is, the worse the function of insulin in real-time glucose regulation is. The temperature of human skin surface usually ranges from 32° to 37°. In this temperature range, the insulin peak action time is relatively long. The lower the temperature is, the longer the insulin peak action time is. Currently, conventional artificial pancreas systems cannot solve the delay between the desired glucose concentration and the insulin peak action time. Therefore, currently conventional artificial pancreas systems cannot real-timely and rapidly adjust the glucose concentration.
WO 2009/066288 A1 discloses a skin adherable device for delivering therapeutic fluid into a body of a patient. The device includes a monitoring apparatus, a dispensing apparatus, and a tip for delivering the therapeutic fluid into the body of the patient and for monitoring bodily analyte in the body of the patient. The dispensing apparatus may continuously deliver the therapeutic fluid to the body of the patient and the monitoring apparatus may continuously monitor bodily analytes of the patient.
US 2008/086042 A1 discloses systems and methods of use for continuous analyte measurement of a host's vascular system. In some embodiments, a continuous glucose measurement system includes a vascular access device, a sensor and sensor electronics, the system being configured for insertion into communication with a host's circulatory system.

### SUMMARY

Regarding the above-mentioned shortcomings of the prior art, an object of the present disclosure is to provide a single needle integrated artificial pancreas for solving the problems that when a patient uses the current conventional artificial pancreas, as a glucose sensor and an indwelling cannula are required to be respectively placed in the subcutaneous tissue, multiple punctures are required. As a result, the installation of the device is complex and the chance of infection is high.

In order to achieve the above-mentioned purposes and other related purposes, the present disclosure provides a single needle integrated artificial pancreas, including: a fluid reservoir unit configured to store insulin; a fluid driving unit connected to the fluid reservoir unit and configured to deliver the insulin stored in the fluid reservoir unit to the subcutaneous tissue of a patient; an indwelling unit connected to the fluid reservoir unit, including a glucose sensor, an indwelling cannula, a groove puncture needle and an installation device; wherein the glucose sensor is an electrode sensor comprising an enzyme layer, and configured to monitor, in real time, a change of glucose and produce effective glucose signals based on the change of the glucose; and a glucose monitoring and insulin delivery control unit, which are connected to the fluid reservoir unit, the drug delivery system and the indwelling unit, and configured to process the effective glucose signals output by the glucose sensor, and to control the fluid reservoir unit, the fluid driving unit and the indwelling unit; wherein the electrode sensor and the indwelling cannula are simultaneously implanted in the same subcutaneous tissue in the patient via the installation device with the aid of the groove puncture needle; wherein the single needle integrated artificial pancreas further includes a skin temperature automatic adjusting device configured to heat the skin tissue where the glucose sensor and the indwelling cannula are implanted; wherein the electrode sensor together with the indwelling cannula is located in a groove of the groove puncture needle; wherein a distance between an end of the indwelling cannula and an end of the electrode sensor ranges from 1mm to 8mm.

Optionally, the electrode sensor is integrated in the outer surface of the indwelling cannula.

Optionally, the electrode of the electrode sensor is ring-shaped or fan-shaped, and an electrode contact point of the electrode sensor is ring-shaped or fan-shaped.

Optionally, the electrode sensor has an electrode coating structure including an anti-interference layer, the enzyme layer, a regulation layer and a protective layer.

Optionally, the electrode sensor has an electrode coating structure including the enzyme layer, a regulation layer and a protective layer.

Optionally, the electrode sensor has an electrode coating structure including an anti-interference layer, a bonding layer, the enzyme layer, a bonding layer, a regulation layer and a protective layer.

Optionally, the indwelling cannula and the glucose sensor are arranged in parallel and located in the groove of the groove puncture needle.

Optionally, the end of the indwelling cannula and the end of the glucose sensor are both tip shaped.

Optionally, the skin temperature automatic adjusting device includes a skin heating film, a heating module, a temperature control module, a heat preservation module and a temperature detection module.

Optionally, the installation device is an automatic ejecting installation device or a manual installation device.

Optionally, the single needle integrated artificial pancreas further includes a pump base and a controller which is connected to the pump base, wherein the pump base includes the fluid reservoir unit, the fluid driving unit and the indwelling unit, wherein the controller includes the glucose monitoring and insulin delivery control unit.

In comparison with prior art, technique solutions provided by the present disclosure have following advantages.

In the present disclosure, the indwelling cannula and the glucose sensor of the artificial pancreas are implanted in the same place of the subcutaneous tissue, so the chance of patient infection decreases.

Moreover, the single needle integrated artificial pancreas is easy to install and use.

Moreover, the single needle integrated artificial pancreas solves the delay between the desired glucose concentration and the insulin peak action time, real-timely and rapidly adjusts the glucose concentration, and reduces the effect of temperature change on glucose monitoring.

### BRIEF DESCRIPTION OF THE DRAWINGS

Figure 1 illustrates a schematic diagram of a single needle integrated artificial pancreas in this disclosure;
Figure 2 illustrates a schematic diagram of the glucose sensor with an electrode sensor in a single needle integrated artificial pancreas in this disclosure;
Figure 3 illustrates a schematic diagram of the glucose sensor with a fiber optic sensor in a single needle integrated artificial pancreas in a single needle integrated artificial pancreas in this disclosure;
Figure 4 illustrates a schematic diagram of a puncture needle in the indwelling cannula integrated with an electrode sensor in a single needle integrated artificial pancreas in this disclosure;
Figure 5 illustrates a schematic diagram of the electrode's structure of a electrode sensor integrated in the outside surface of the indwelling cannula in a single needle integrated artificial pancreas in this disclosure;
Figure 6 illustrates a schematic diagram of the electrode's structure of a electrode sensor integrated in an outside surface of the indwelling cannula in a single needle integrated artificial pancreas in this disclosure;
Figure 7 illustrates a schematic diagram of the electrode contact point's structure of a electrode sensor integrated in an outside surface of the indwelling cannula in a single needle integrated artificial pancreas in this disclosure;
Figure 8 illustrates a schematic diagram of the electrode contact point's structure of a electrode sensor integrated in an outside surface of the indwelling cannula in a single needle integrated artificial pancreas in this disclosure;
Figure 9 illustrates another schematic diagram of an indwelling cannula and an electrode sensor integrated in an outside surface of the indwelling cannula in puncture needle of a single needle integrated artificial pancreas in this disclosure;
Figure 10 illustrates a schematic diagram of an indwelling cannula and an electrode sensor in puncture needle of a single needle integrated artificial pancreas in this disclosure;
Figure 11 illustrates a schematic diagram of a fiber optic sensor and an indwelling cannula arranged in parallel in puncture needle of a single needle integrated artificial pancreas in this disclosure;
Figure 12 illustrates a schematic diagram of an electrode sensor and an indwelling cannula arranged in parallel in puncture needle of a single needle integrated artificial pancreas in this disclosure;
Figure 13 illustrates a schematic diagram of an electrode sensor, a fiber optic sensor and an indwelling cannula arranged in parallel in puncture needle of a single needle integrated artificial pancreas in this disclosure;
Figure 14A illustrates a schematic structure diagram of a skin temperature automatic adjusting device of a single needle integrated artificial pancreas in this disclosure; and
Figure 14B illustrates a schematic structure diagram of a skin temperature automatic adjusting device of a single needle integrated artificial pancreas in this disclosure.

### Brief description of reference signs

- 1: fluid reservoir unit
- 2: fluid driving unit
- 3: indwelling unit
- 4: glucose monitoring and insulin delivery control unit
- 5: skin temperature automatic adjusting device
- 31: glucose sensor
- 32: indwelling cannula
- 33: puncture needle
- 34: inserter
- 35: electrode sensor
- 36: fiber optic sensor
- 51: skin heating film
- 52: heating module
- 53: temperature control module
- 54: heat preservation module
- 55: temperature detection module
- 331: steel puncture needle
- 332: groove puncture needle
- 351: counter electrode
- 352: working electrode
- 353: reference electrode
- 354: electrode contact point
- 361: acquisition area of fiber signal
- 362: transmission area of fiber signal

### DETAILED DESCRIPTION OF THE DISCLOSURE

The embodiments of the present disclosure are described in the following through specific examples, and those skilled in the art can easily understand other advantages and effects of the present disclosure according to the content disclosed in the specification. The present disclosure may also be implemented or applied through other different specific examples, and various modifications and variations may be made to the details in the specification on the basis of different opinions and applications without departing from the spirit of the present disclosure.

Referring to the figures, it should be noted that, the drawing provided in this embodiment exemplary shows the basic concept of the present disclosure, so that components related to the present disclosure are merely shown in the drawings and are not drawn based on the actual number, shape or size in implementation. The actual shape, number and proportion of each component in implementation may vary arbitrarily, and the layout of the components may also be more complex.

The embodiments of the present disclosure will be described in detail in conjunction with the accompanying drawings.

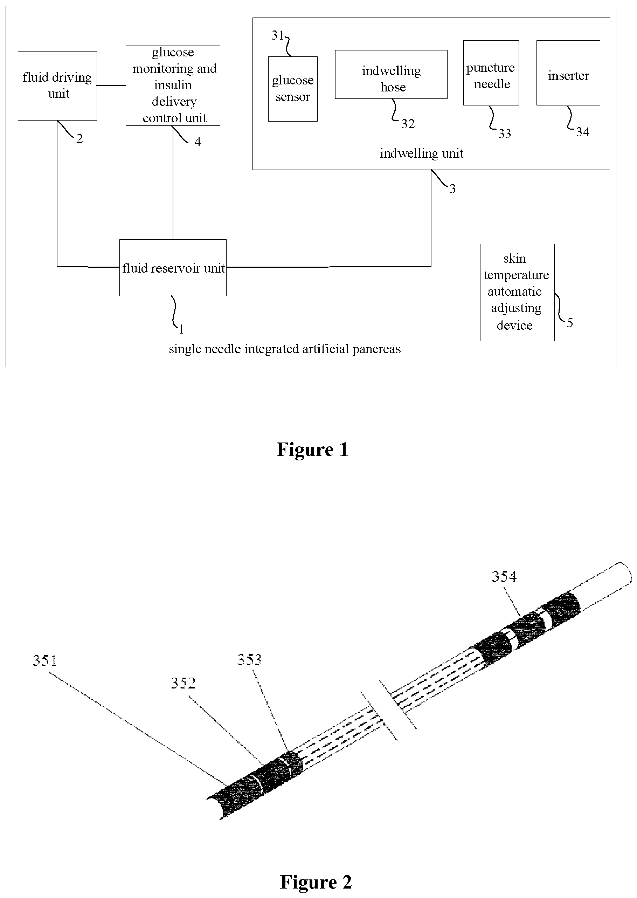

The present disclosure provides a single needle integrated artificial pancreas, referring to FIG. 1, a schematic diagram of a single needle integrated artificial pancreas is illustrated, the single needle integrated artificial pancreas includes: a fluid reservoir unit 1, a fluid driving unit 2, an indwelling unit 3, a glucose monitoring and insulin delivery control unit 4, and a skin temperature automatic adjusting device 5. The indwelling unit 3 includes a glucose sensor 31, an indwelling cannula 32, a puncture needle 33, and an inserter 34. The puncture needle 33 is a steel puncture needle 331 or a groove puncture needle 332. The inserter is an automatic ejecting inserter or a manual inserter. The single needle integrated artificial pancreas further includes a pump base and a controller connected to the pump base, wherein the pump base includes the fluid reservoir unit, the fluid driving unit, and the indwelling unit, the controller includes the glucose monitoring and insulin delivery control unit.

The fluid reservoir unit 1 is configured to store an insulin;

The fluid driving unit 2 is connected to the fluid reservoir unit 1 and configured to deliver the insulin stored in the fluid reservoir unit 1 to the subcutaneous tissue of a patient;

The indwelling unit 3 is connected to the fluid reservoir unit 1, and the indwelling unit 3 includes the glucose sensor 31, the indwelling cannula 32, the puncture needle 33 and the inserter 34.

The glucose sensor 31 is configured to real-timely monitor a change of the glucose, produce effective glucose signals based on the change of the glucose, and output the effective glucose signals. In some embodiments, the glucose sensor may be an electrode sensor 35, a fiber optic sensor 36, or a combination thereof. The glucose sensor is implanted in the subcutaneous tissue, when it is used to monitor the glucose.

The glucose sensor 31 may be an electrode sensor which monitors the glucose by current signal. The glucose electrode sensor mainly includes an enzyme layer, and a Clark electrode or a hydrogen peroxide electrode. The glucose may occur an oxidation reaction under the catalysis of glucose oxidase, consume oxygen, and produce glucolactone and hydrogen peroxide. The glucose oxidase is fixed on the surface of the platinum electrode by using physical adsorption method by semipermeable membrane, whose activity depends on the oxygen concentration around it. The reaction between the glucose and the glucose oxidase produces two electrons and two protons. The glucose oxidase reduced reacts with oxygen, electron and proton which are enclosing the glucose oxidase, and produces hydrogen peroxide and the glucose oxidase which may react with glucose. The higher the glucose concentration is, the more oxygen is consumed, and the more hydrogen peroxide is produced; the lower the glucose concentration is, it is opposite. Therefore, the consumption of oxygen and the production of hydrogen peroxide may both be detected by the platinum electrode, and which are used as a method to detect the glucose. The glucose sensor may be a three electrode system or a two electrode system, wherein the three electrode system includes a counter electrode, a reference electrode, and at least one working electrode. The present disclosure has two cases based on the number of the working electrode:
1) Single working electrode, there is only one working electrode;
2) Double working electrode, there is two working electrodes, one is called "working electrode", which is configured to occur electro oxidation reaction or electro reduction reaction with the materials detected, and produce electrical signal; the other is called "auxiliary electrode", which is configured to detect the response signal of the interferent and the background solution.

The reference electrode in three electrode system is configured to effectively control detection electric potential, and prevent electric potential drifting. For the two electrode system, its structure is simpler, and its production cost is lower. Referring to FIG. 2, a schematic diagram of the glucose sensor with an electrode sensor is illustrated. In some embodiments, the glucose sensor uses the electrode sensor 35, wherein the electrode sensor 35 is a three electrode system, which includes a counter electrode 351, a working electrode 352, a reference electrode 353 and an electrode contact point (PAD) 354.

An electrode coating structure of the electrode sensor in the glucose sensor includes: (1) the electrode sensor has an electrode coating structure including an anti-interference layer, an enzyme layer, a regulation layer and a protective layer; (2) the electrode sensor has an electrode coating structure including an enzyme layer, a regulation layer, and a protective layer; (3) the electrode sensor has an electrode coating structure including an anti-interference layer, a bonding layer, an enzyme layer, a bonding layer, a regulation layer, and a protective layer. Using such electrode coating structure of the electrode sensor may improve the accuracy and specificity of the glucose monitoring, and such electrode coating structure of the electrode sensor has good stability in vivo or in vitro.

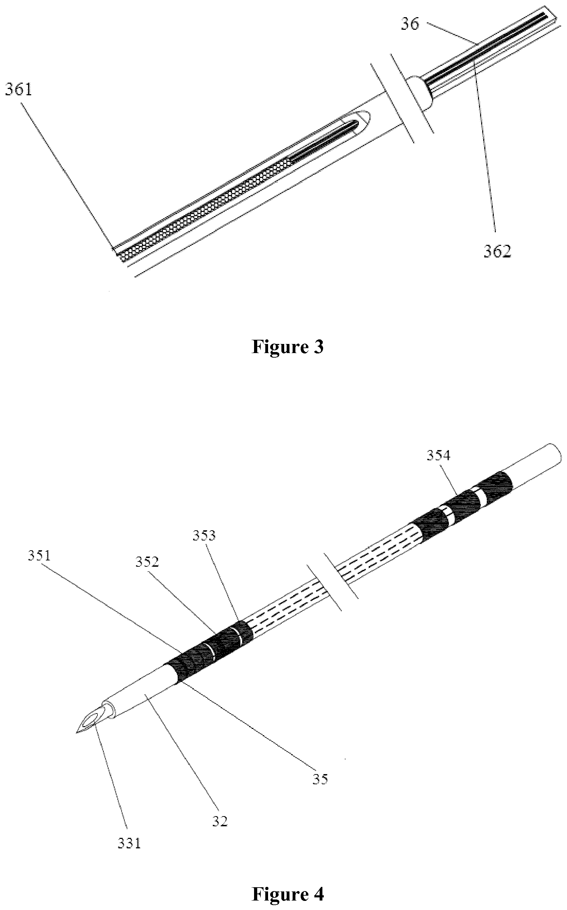

The glucose sensor 31 may be a fiber optic sensor 36 which monitors the glucose by optical signal, wherein the fiber optic sensor includes the fiber optic biosensor based on fluorescence. The glucose fiber optic sensor produces fluorescence when the semiconductor quantum dots are irradiated by light of specific wavelengths transmitted by micro optical fiber. Reaction between the glucose and the glucose oxidase produces the hydrogen peroxide which may reduce the intensity of fluorescence, and even quench the fluorescence. The degree of light intensity decreasing has a certain linear relation with the glucose concentration. By the linear relation, the glucose concentration is monitored. Referring to FIG. 3, a schematic diagram of the glucose sensor with a fiber optic sensor is illustrated. In the embodiment, the glucose sensor uses the fiber optic sensor 36, wherein the fiber optic sensor 36 includes an acquisition area of fiber signal 361 and a transmission area of fiber signal 362. The indwelling cannula 32 is configured to deliver the insulin in the fluid reservoir unit 1, wherein the indwelling cannula 32 is connected to the fluid reservoir unit 1.

The puncture needle 33 is configured to puncture the skin of the patient. In some embodiments, the puncture needle 33 is the steel puncture needle 331 or the groove puncture needle 332.

The inserter 34 is configured to simultaneously implant the glucose sensor 31 and the indwelling cannula 32 in the same subcutaneous tissue in the patient with the aid of the puncture needle 33. The inserter 34 may simultaneously implant the glucose sensor 31 and the indwelling cannula 32 in the same subcutaneous tissue in the patient, so the installation is easy, and the chance of patient infection decreases. When the glucose sensor 31 and the indwelling cannula 32 are integrated, and the puncture needle 33 is the steel puncture needle 331 or the groove puncture needle 332. That is, the indwelling cannula 32 and the glucose sensor 31 which is integrated in the outer surface of the indwelling cannula 32 are implanted in the subcutaneous tissue in the patient by using the steel puncture needle 331 or the groove puncture needle 332 located inside the indwelling cannula 32. When the glucose sensor 31 and the indwelling cannula 32 are arranged in parallel, the puncture needle 33 is the groove puncture needle 332.

In some embodiments, simultaneously implanting the glucose sensor 31 and the indwelling cannula 32 in the same subcutaneous tissue in the patient may be implemented in the following embodiments:

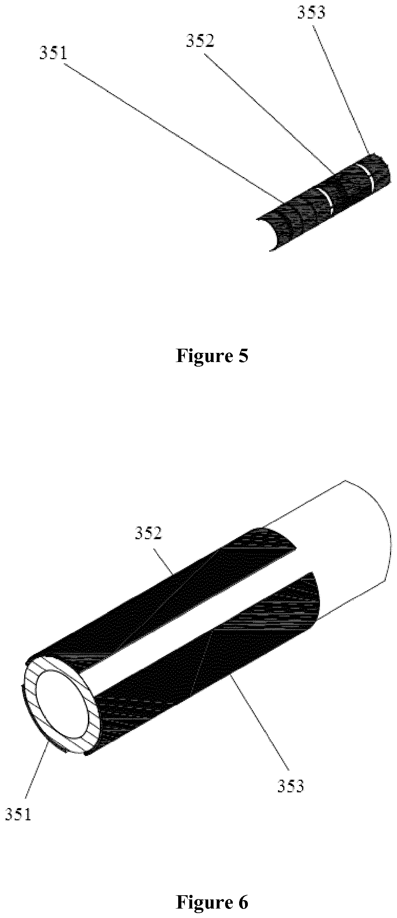

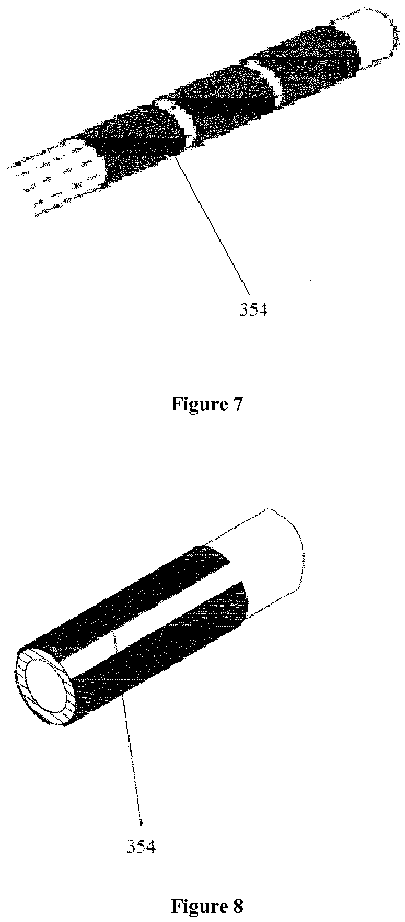

The first embodiment, referring to FIG. 4, a schematic diagram of a puncture needle in the indwelling cannula integrated with an electrode sensor is illustrated. In the first embodiment, the glucose sensor 31 uses the electrode sensor 35; the puncture needle 33 is the steel puncture needle 331. At the moment, the steel puncture needle 331 is located inside the indwelling cannula 32. The electrode sensor 35 is integrated in the outer surface of the indwelling cannula 32, the electrode of the electrode sensor 35 is ring-shaped or fan-shaped, and the PAD 354 of the electrode sensor 35 is ring-shaped or fan-shaped. The electrode of the electrode sensor 35 is ring-shaped or fan-shaped, referring to FIG. 5, a schematic diagram of a ring-shaped electrode's structure of a electrode sensor integrated in an outside surface of the indwelling cannula is illustrated; referring to FIG. 6, a schematic diagram of a fan-shaped electrode's structure of a electrode sensor integrated in the outer surface of the indwelling cannula, and the PAD 354 of the electrode sensor is ring-shaped or fan-shaped is illustrated; referring to FIG. 7, a schematic diagram of a fan-shaped PAD's structure of a electrode sensor integrated in the outer surface of the indwelling cannula is illustrated; referring to FIG. 8, a schematic diagram of a ring-shaped PAD's structure of a electrode sensor integrated in the outer surface of the indwelling cannula is illustrated. The glucose sensor 31 and the indwelling cannula 32 integrated are implanted in the subcutaneous tissue in the patient by the steel puncture needle 331 in the indwelling cannula. In order to reduce the puncture resistance in the process of implantation, the end of the indwelling cannula is tip shape.

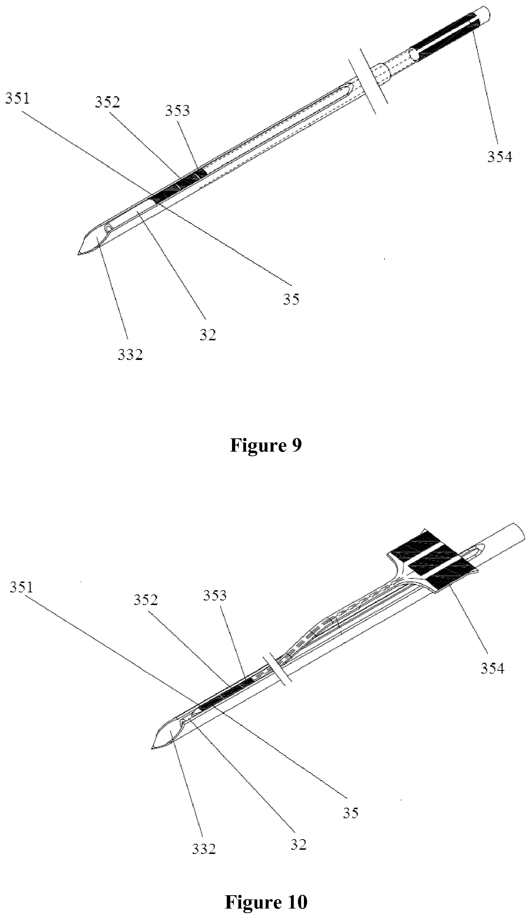

The second embodiment, referring to FIG. 9, a schematic diagram of an indwelling cannula and an electrode sensor integrated in the outer surface of the indwelling cannula in puncture needle is illustrated. In the second embodiment, the glucose sensor 31 uses the electrode sensor 35; the puncture needle 33 is the groove puncture needle 332. At the moment, the glucose sensor 31 and the indwelling cannula 32 are located in the groove of the groove puncture needle 332, and they are implanted in the subcutaneous tissue in the patient by using the groove puncture needle 332. The electrode of the electrode sensor 35 is ring-shaped or fan-shaped; the PAD 354 of the electrode sensor 35 is ring-shaped or fan-shaped. In order to eliminate the effect of insulin injection on glucose monitoring, the distance between the end of the indwelling cannula 32 and the end of the glucose sensor 31 ranges from 1mm to 8mm. In first embodiment and second embodiment, the distance between the end of the indwelling cannula 32 and the end of the glucose sensor 31 ranges from 2mm to 4mm.

The third embodiment, referring to FIG. 10, a schematic diagram of an indwelling cannula and an electrode sensor arranged in parallel in puncture needle is illustrated. In the third embodiment, the glucose sensor 31 uses the electrode sensor 35, wherein the electrode sensor 35 is a three electrode system, which includes the counter electrode 351, the working electrode 352, the reference electrode 353 and the electrode contact point (PAD) 354; the puncture needle 33 is the groove puncture needle 332. At the moment, the glucose sensor 31 and the indwelling cannula 32 are arranged in parallel in the groove of the groove puncture needle 332, and they are implanted in the subcutaneous tissue in the patient by using the groove puncture needle 332. When the glucose sensor 31 and the indwelling cannula 32 are arranged in parallel, in order to reduce the puncture resistance in the process of implantation, the end of the indwelling cannula 32 and the glucose sensor 31 are both tip shape. Moreover, in order to eliminate the effect of insulin injection on glucose monitoring, the distance between the end of the indwelling cannula 32 and the end of the glucose sensor 31 ranges from 1mm to 8mm. In some embodiments, the distance between the end of the indwelling cannula 32 and the end of the electrode sensor 35 ranges from 2mm to 4mm.

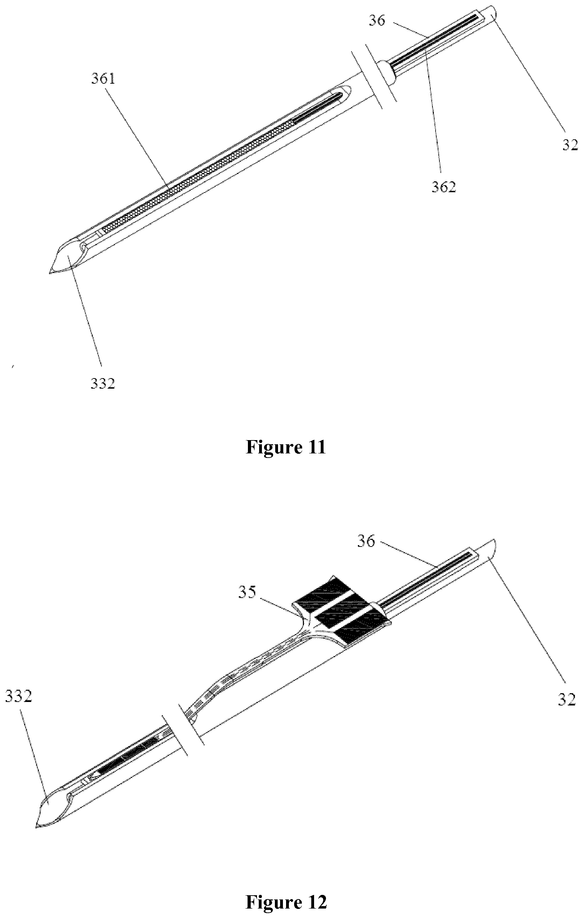

The fourth embodiment, referring to FIG. 11, a schematic diagram of an indwelling cannula 32 and a fiber optic sensor 36 arranged in parallel in puncture needle 33 is illustrated. In the fourth embodiment, the glucose sensor uses the fiber optic sensor 36, wherein the fiber optic sensor 36 includes the acquisition area of fiber signal 361 and the transmission area of fiber signal 362; the puncture needle 33 is the groove puncture needle 332. At the moment, the fiber optic sensor 36 and the indwelling cannula 32 are arranged in parallel in the groove of the groove puncture needle 332. In some embodiments, the distance between the end of the indwelling cannula 32 and the end of the fiber optic sensor 36 ranges from 2mm to 4mm.

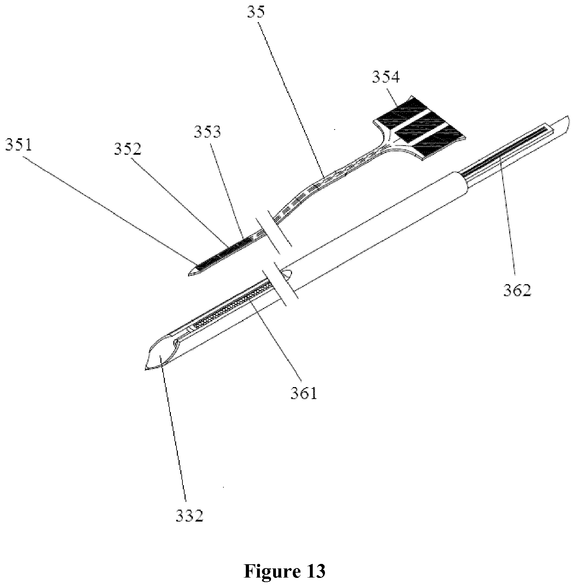

The fifth embodiment, referring to FIG. 12 and FIG. 13, a schematic diagram of an indwelling cannula 32 and a glucose sensor 31 arranged in parallel in puncture needle 33 are illustrated. In the fifth embodiment, the glucose sensor 31 uses the electrode sensor 35 and the fiber optic sensor 36; the puncture needle 33 is the groove puncture needle 332. At the moment, the electrode sensor 35, the fiber optic sensor 36 and the indwelling cannula 32 are arranged in parallel in the groove of the groove puncture needle 332. Simultaneously using the electrode sensor 35 and the fiber optic sensor 36 to monitor glucose, and using special algorithm may improve the reliability and accuracy of glucose monitoring.

In some embodiments, the inserter of the single needle integrated artificial pancreas may be an automatic ejecting inserter or a manual inserter. There are two installation method in some embodiments, one is that installing the automatic ejecting inserter in artificial pancreas, the glucose sensor 31 and the indwelling cannula 32 are simultaneously implanted in the same subcutaneous tissue in the patient via the automatic ejecting inserter under the aid of the puncture needle 33; the other is that the glucose sensor 31 and the indwelling cannula 32 are simultaneously implanted in the same subcutaneous tissue in the patient via the manual inserter under the aid of the puncture needle 33. After the inserter completes the installing of the glucose sensor 31 and the indwelling cannula 32, the puncture needle 33 is pulled out.

The glucose monitoring and insulin delivery control unit 4 is configured to process a useful blood sugar signal output by the glucose sensor 31, and to control the fluid reservoir unit 1, the drug delivery system 2 and the indwelling unit 3, wherein the glucose monitoring and insulin delivery control unit 4 is connected to the fluid reservoir unit 1, the drug delivery system 2 and the indwelling unit 3. The glucose monitoring and insulin delivery control unit 4 may real-timely monitor and detect the glucose concentration, so the patient and the paramedic may real-timely know the change of the glucose concentration, and make a response.

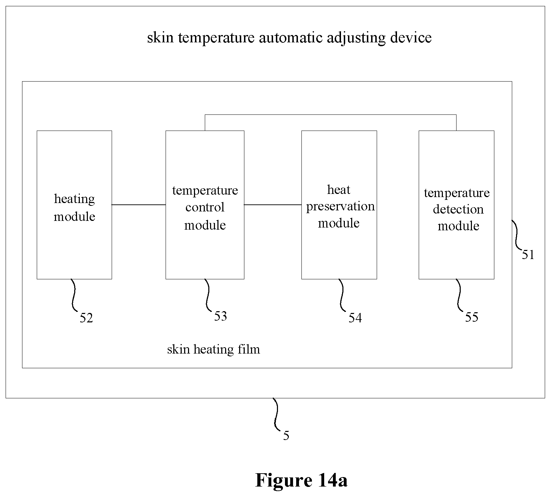

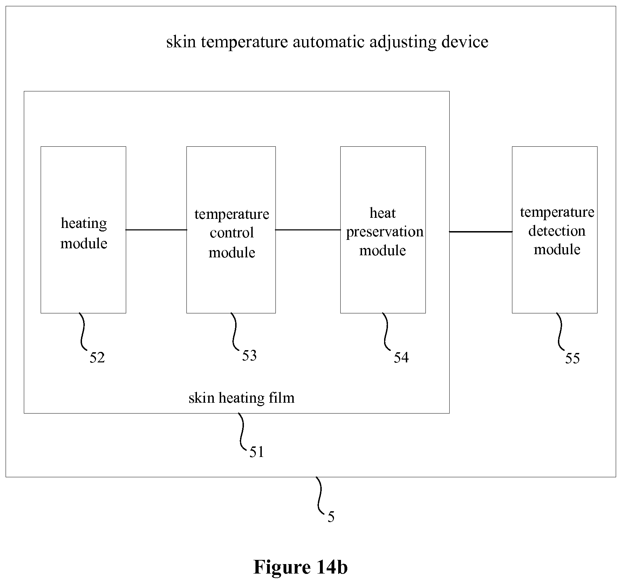

The skin temperature automatic adjusting device 5 is configured to heat the skin tissue at which the glucose sensor 31 and the indwelling cannula 32 are implanted. The skin temperature automatic adjusting device 5 is stuck on surface of the skin tissue at which the glucose sensor 31 and the indwelling cannula 32 are implanted. After the skin temperature automatic adjusting device 5 is electrified, it may produce a desired temperature which may reduce the effect of temperature change on glucose monitoring, and reduce the delay of the insulin peak action time. Referring to FIG. 14a and FIG. 14b, a schematic diagram of a skin temperature automatic adjusting device 5 are illustrated. The skin temperature automatic adjusting device 5 includes a skin heating film 51, a heating module 52, a temperature control module 53, a heat preservation module 54 and a temperature detection module 55.

### The skin heating film 51

The heating module 52 is configured to heat the skin tissue at which the glucose sensor 31 and the indwelling cannula 32 are implanted to 32°C∼45°C, wherein the heating module 52 is located in the skin heating film 51. In some embodiments, the heating module 52 may be a fast heating integrated circuit which includes a heating element, a power, a temperature sampling element and a switching element.

The temperature control module 53 is configured to regulate heating parameter and control the temperature range, wherein the temperature control module 53 is located in the skin heating film 51 and connected to the heating module 52. In some embodiments, the temperature control module 53 may be a temperature control circuit which includes a temperature sensor, a relay and a temperature regulation circuit, and control the temperature of skin tissue at 32°C∼45°C.

The heat preservation module 54 is configured to preserve the skin tissue at which the glucose sensor 31 and the indwelling cannula 32 are implanted at 32°C∼45°C, wherein the heat preservation module 54 is located in the skin heating film 51 and connected to the temperature control module 53. In some embodiments, the heat preservation module 54 may be a heat preservation circuit, the temperature is set at 32°C∼45°C, wherein the heat preservation circuit includes a temperature sensor, an operational amplifier, a comparator and a heating unit.

The temperature detection module 55 is configured to detect the skin tissue at which the glucose sensor 31 and the indwelling cannula 32 are implanted. In some embodiments, the temperature detection module 55 may be a temperature detection circuit which includes a temperature sensor, a singlechip and a temperature detection circuit unit.

In some embodiments, the heating module 52, the temperature control module 53, the heat preservation module 54 and the temperature detection module 55 may be located in the skin heating film 51, and constitute the skin temperature automatic adjusting device 5, wherein the temperature detection module 55 is connected to the temperature control module 53. In some embodiments, the heating module 52, the temperature control module 53 and the heat preservation module 54 may be located in the skin heating film 51, connecting the temperature detection module 55 and the skin heating film 51, which constitutes the skin temperature automatic adjusting device 5.

The skin temperature automatic adjusting device 5, the fluid reservoir unit 1, the fluid driving unit 2, the indwelling unit 3, and the glucose monitoring and insulin delivery control unit 4 complete the glucose monitoring and insulin injection together. In the process, the skin is heated by the skin temperature automatic adjusting device 5, which may solve the delay between the desired glucose concentration and the insulin peak action time, and reduce the effect of temperature change on glucose monitoring.

The single needle integrated artificial pancreas according to the present disclosure provides the inserter which is configured to simultaneously implant the glucose sensor and the indwelling cannula in the same subcutaneous tissue in the patient under the aid of the puncture needle, so the chance of patient infection decreases, and the installation of the single needle integrated artificial pancreas is easy; while the single needle integrated artificial pancreas according to the present disclosure provides the skin temperature automatic adjusting device which may heat the skin, so which may solve the delay between the desired glucose concentration and the insulin peak action time, and reduce the effect of temperature change on glucose monitoring.

In summary, the present disclosure overcomes the various shortcomings of the current technology and has high industrial utilization value.

## Claims

1. A single needle integrated artificial pancreas, comprising:
a fluid reservoir unit (1) configured to store insulin;
a fluid driving unit (2) connected to the fluid reservoir unit (1) and configured to deliver the insulin stored in the fluid reservoir unit (1) to the subcutaneous tissue of a patient;
an indwelling unit (3) connected to the fluid reservoir unit (1), comprising a glucose sensor (31), an indwelling cannula (32), a groove puncture needle (332) and
an inserter
(34), wherein the glucose sensor (31) is an electrode sensor (35) comprising an enzyme layer, and configured to monitor in real time a change of glucose and produce effective glucose signals based on the change of the glucose; and a glucose monitoring and insulin delivery control unit (4), which is connected to the fluid reservoir unit (1), the fluid driving unit (2) and the indwelling unit (3), and configured to process the effective glucose signals output by the glucose sensor (31), and to control the fluid reservoir unit (1), the fluid driving unit (2) and the indwelling unit (3);
wherein the electrode sensor (35) and the indwelling cannula (32) are simultaneously implanted in a same subcutaneous tissue in the patient via the
inserter
(34) with the aid of the groove puncture needle (332);
**characterized in that** the single needle integrated artificial pancreas further comprises a skin temperature automatic adjusting device (5) configured to heat the skin tissue where the electrode sensor (35) and the indwelling cannula (32) are implanted;
wherein the electrode sensor (35) together with the indwelling cannula (32) is located in a groove of the groove puncture needle (332);
wherein a distance between an end of the indwelling cannula (32) and an end of the electrode sensor (35) ranges from 1mm to 8mm.

2. The single needle integrated artificial pancreas according to claim 1, wherein the electrode sensor (35) is integrated in the outer surface of the indwelling cannula (32).

3. The single needle integrated artificial pancreas according to claim 2, wherein the electrode (351, 352, 353) of the electrode sensor (35) is ring-shaped or fan-shaped, and an electrode contact point (354) of the electrode sensor (35) is ring-shaped or fan-shaped.

4. The single needle integrated artificial pancreas according to claim 1, wherein the electrode sensor (35) has an electrode coating structure comprising an anti-interference layer, the enzyme layer, a regulation layer and a protective layer.

5. The single needle integrated artificial pancreas according to claim 1, wherein the electrode sensor (35) has an electrode coating structure comprising the enzyme layer, a regulation layer and a protective layer.

6. The single needle integrated artificial pancreas according to claim 1, wherein the electrode sensor (35) has an electrode coating structure comprising an anti-interference layer, a bonding layer, the enzyme layer, a bonding layer, a regulation layer and a protective layer.

7. The single needle integrated artificial pancreas according to claim 1, wherein the indwelling cannula (32) and the electrode sensor (35) are arranged in parallel.

8. The single needle integrated artificial pancreas according to claim 1, wherein the end of the indwelling cannula (32) and the end of the electrode sensor (35) are both tip shaped.

9. The single needle integrated artificial pancreas according to claim 1, wherein the skin temperature automatic adjusting device (5) comprises a skin heating film (51), a heating module (52), a temperature control module (53), a heat preservation module (54) and a temperature detection module (55).

10. The single needle integrated artificial pancreas according to claim 1, wherein the
inserter
(34) is an automatic ejecting installation device or a manual installation device.

11. The single needle integrated artificial pancreas according to claim 1, further comprising a pump base and a controller connected to the pump base, wherein the pump base comprises the fluid reservoir unit (1), the fluid driving unit (2) and the indwelling unit (3), wherein the controller comprises the glucose monitoring and insulin delivery control unit (4).

## Patentansprüche

1. Einnadelig integrierte künstliche Bauchspeicheldrüse, umfassend:
ein Fluidreservoir (1), das konfiguriert ist, um Insulin zu speichern;
eine Fluidantriebseinheit (2), die mit dem Fluidreservoir (1) verbunden und konfiguriert ist, um das in dem Fluidreservoir (1) gespeicherte Insulin an das subkutane Gewebe eines Patienten zu liefern;
eine Dauereinheit (3), die mit dem Fluidreservoir (1) verbunden ist, umfassend einen Glukosesensor (31), einen Dauerkatheter (32), eine Nutpunktionsnadel (332) und eine Einführvorrichtung (34), wobei der Glukosesensor (31) ein Elektrodensensor (35) ist, umfassend eine Enzymschicht, und konfiguriert ist, um in Echtzeit eine Änderung von Glukose zu überwachen und effektiv Glukosesignale zu erzeugen, basierend auf der Änderung der Glukose; und
eine Glukoseüberwachungs- und Insulinliefersteuerungseinheit (4), die mit dem Fluidreservoir (1), der Fluidantriebseinheit (2) und der Dauereinheit (3) verbunden ist und konfiguriert ist, um die effektiven Glukosesignale, die von dem Glukosesensor (31) ausgegeben werden, zu verarbeiten und um die Fluidreservoireinheit (1), die Fluidantriebseinheit (2) und die Dauereinheit (3) zu steuern;
wobei der Elektrodensensor (35) und der Dauerkatheter (32) gleichzeitig in ein gleiches subkutanes Gewebe in dem Patienten eingesetzt werden über die Einführvorrichtung (34) mit Hilfe der Nutpunktionsnadel (332);
**dadurch gekennzeichnet, dass** die einnadelig integrierte künstliche Bauchspeicheldrüse ferner eine automatische Hauttemperaturanpassungsvorrichtung (5) umfasst, die konfiguriert ist, um das Hautgewebe dort zu erwärmen, wo der Elektrodensensor (35) und der Dauerkatheter (32) eingesetzt sind;
wobei sich der Elektrodensensor (35) zusammen mit dem Dauerkatheter (32) in einer Nut der Nutpunktionsnadel (332) befindet;
wobei eine Entfernung zwischen einem Ende des Dauerkatheters (32) und einem Ende des Elektrodensensors (35) von 1 mm bis zu 8 mm reicht.

2. Einnadelig integrierte künstliche Bauchspeicheldrüse nach Anspruch 1, wobei der Elektrodensensor (35) in die Außenfläche des Dauerkatheters (32) integriert ist.

3. Einnadelig integrierte künstliche Bauchspeicheldrüse nach Anspruch 2, wobei die Elektrode (351, 352, 353) des Elektrodensensors (35) ringförmig oder fächerförmig ist und eine Elektrodenkontaktstelle (354) des Elektrodensensors (35) ringförmig oder fächerförmig ist.

4. Einnadelig integrierte künstliche Bauchspeicheldrüse nach Anspruch 1, wobei der Elektrodensensor (35) eine Elektrodenbeschichtungsstruktur hat, umfassend eine Antistörschicht, die Enzymschicht, eine Regulationsschicht und eine Schutzschicht.

5. Einnadelig integrierte künstliche Bauchspeicheldrüse nach Anspruch 1, wobei der Elektrodensensor (35) eine Elektrodenbeschichtungsstruktur hat, umfassend die Enzymschicht, eine Regulationsschicht und eine Schutzschicht.

6. Einnadelig integrierte künstliche Bauchspeicheldrüse nach Anspruch 1, wobei der Elektrodensensor (35) eine Elektrodenbeschichtungsstruktur hat, umfassend eine Antistörschicht, eine Verbindungsschicht, die Enzymschicht, eine Verbindungsschicht, eine Regulationsschicht und eine Schutzschicht.

7. Einnadelig integrierte künstliche Bauchspeicheldrüse nach Anspruch 1, wobei der Dauerkatheter (32) und der Elektrodensensor (35) parallel angeordnet sind.

8. Einnadelig integrierte künstliche Bauchspeicheldrüse nach Anspruch 1, wobei das Ende des Dauerkatheters (32) und das Ende des Elektrodensensors (35) beide spitz geformt sind.

9. Einnadelig integrierte künstliche Bauchspeicheldrüse nach Anspruch 1, wobei die automatische Hauttemperaturanpassungsvorrichtung (5) einen Hauterwärmungsfilm (51), ein Erwärmungsmodul (52), ein Temperatursteuerungsmodul (53), ein Wärmebewahrungsmodul (54) und ein Temperaturerfassungsmodul (55) umfasst.

10. Einnadelig integrierte künstliche Bauchspeicheldrüse nach Anspruch 1, wobei die Einführvorrichtung (34) eine automatische Ausgabeinstallationsvorrichtung oder eine manuelle Installationsvorrichtung ist.

11. Einnadelig integrierte künstliche Bauchspeicheldrüse nach Anspruch 1, ferner umfassend eine Pumpbasis und eine Steuerung, die mit der Pumpbasis verbunden ist, wobei die Pumpbasis die Fluidreservoireinheit (1), die Fluidantriebseinheit (2) und die Dauereinheit (3) umfasst, wobei die Steuerung die Glukoseüberwachungs- und Insulinliefersteuerungseinheit (4) umfasst.

## Revendications

1. Pancréas artificiel intégré à aiguille unique, comprenant :
une unité à réservoir de fluide (1) configurée pour stocker de l'insuline ;
une unité d'entraînement de fluide (2) raccordée à l'unité à réservoir de fluide (1) et configurée pour fournir l'insuline stockée dans l'unité à réservoir de fluide (1) au tissu sous-cutané d'un patient ;
une unité à demeure (3) raccordée à l'unité à réservoir de fluide (1), comprenant un capteur de glucose (31), une canule à demeure (32), une aiguille pour ponction à rainure (332) et un instrument d'introduction (34), dans lequel le capteur de glucose (31) est un capteur à électrode (35) comprenant une couche d'enzyme, et configuré pour surveiller en temps réel un changement de glucose et produire des signaux de glucose efficace sur la base du changement du glucose ; et
une unité de surveillance de glucose et de commande d'administration d'insuline (4), qui est connectée à l'unité à réservoir de fluide (1), l'unité d'entraînement de fluide (2) et l'unité à demeure (3), et configurée pour traiter les signaux de glucose efficace produits par le capteur de glucose (31), et pour commander l'unité à réservoir de fluide (1), l'unité d'entraînement de fluide (2) et l'unité à demeure (3) ;
dans lequel le capteur à électrode (35) et la canule à demeure (32) sont simultanément implantés dans un même tissu sous-cutané chez le patient par l'intermédiaire de l'instrument d'introduction (34) à l'aide de l'aiguille pour ponction à rainure (332) ;
**caractérisé en ce que** le pancréas artificiel intégré à aiguille unique comprend en outre un dispositif d'ajustement automatique de température de peau (5) configuré pour chauffer le tissu de peau où le capteur à électrode (35) et la canule à demeure (32) sont implantés ;
dans lequel le capteur à électrode (35) conjointement avec la canule à demeure (32) est situé dans une rainure de l'aiguille pour ponction à rainure (332) ;
dans lequel une distance entre une extrémité de la canule à demeure (32) et une extrémité du capteur à électrode (35) varie de 1 mm à 8 mm.

2. Pancréas artificiel intégré à aiguille unique selon la revendication 1, dans lequel le capteur à électrode (35) est intégré dans la surface extérieure de la canule à demeure (32).

3. Pancréas artificiel intégré à aiguille unique selon la revendication 2, dans lequel l'électrode (351, 352, 353) du capteur à électrode (35) présente une forme annulaire ou une forme en éventail, et un point de contact d'électrode (354) du capteur à électrode (35) présente une forme annulaire ou une forme en éventail.

4. Pancréas artificiel intégré à aiguille unique selon la revendication 1, dans lequel le capteur à électrode (35) possède une structure de revêtement d'électrode comprenant une couche anti-interférence, la couche d'enzyme, une couche de régulation et une couche protectrice.

5. Pancréas artificiel intégré à aiguille unique selon la revendication 1, dans lequel le capteur à électrode (35) possède une structure de revêtement d'électrode comprenant la couche d'enzyme, une couche de régulation et une couche protectrice.

6. Pancréas artificiel intégré à aiguille unique selon la revendication 1, dans lequel le capteur à électrode (35) possède une structure de revêtement d'électrode comprenant une couche anti-interférence, une couche de liaison, la couche d'enzyme, une couche de liaison, une couche de régulation et une couche protectrice.

7. Pancréas artificiel intégré à aiguille unique selon la revendication 1, dans lequel la canule à demeure (32) et le capteur à électrode (35) sont agencés en parallèle.

8. Pancréas artificiel intégré à aiguille unique selon la revendication 1, dans lequel l'extrémité de la canule à demeure (32) et l'extrémité du capteur à électrode (35) présentent toutes les deux une forme de pointe.

9. Pancréas artificiel intégré à aiguille unique selon la revendication 1, dans lequel le dispositif d'ajustement automatique de température de peau (5) comprend un film de chauffage de peau (51), un module de chauffage (52), un module de commande de température (53), un module de préservation de chaleur (54) et un module de détection de température (55).

10. Pancréas artificiel intégré à aiguille unique selon la revendication 1, dans lequel l'instrument d'introduction (34) est un dispositif d'installation à éjection automatique ou un dispositif d'installation manuel.

11. Pancréas artificiel intégré à aiguille unique selon la revendication 1, comprenant en outre une base de pompe et un dispositif de commande connecté à la base de pompe, dans lequel la base de pompe comprend l'unité à réservoir de fluide (1), l'unité d'entraînement de fluide (2) et l'unité à demeure (3), dans lequel le dispositif de commande comprend l'unité de surveillance de glucose et de commande d'administration d'insuline (4).
